# EUROPEAN PATENT APPLICATION

(11) **EP 3 252 702 A1**
(43) Date of publication of application: **06.12.2017**
(21) Application number: 15880105.0
(22) Date of filing: 27.11.2015
(51) Int. Cl.: G06Q 50/22, A61B 5/00

(54) **EXAMINATION WORK SUPPORT SYSTEM**

(30) Priority: 28.01.2015 JP 2015014646
(71) Applicant: OLYMPUS CORPORATION, Hachioji-shi Tokyo 192-8507 (JP)
(72) Inventor: SASAKI Akinori, Tokyo 192-8507 (JP)
(74) Representative: Gunzelmann, Rainer
(86) International application number: PCT/JP2015/083441
(87) International publication number: WO 2016/121213

(57) **Abstract**

An examination item management unit 14 manages an examination item and the maximum number of modalities that can acquire an examination order including the examination item in association with each other. An order retrieval unit 18 extracts an examination order to be transmitted from a plurality of examination orders. When the number of the modalities that have acquired an examination order reaches the maximum number that can acquire the examination order, the order retrieval unit 18 does not extract the examination order as an examination order to be transmitted to the modality; on the other hand, when the number of the modalities that have acquired an examination order is less than the maximum number, the order retrieval unit 18 extracts the examination order as an examination order to be transmitted to the modality.

## Description

### [TECHNICAL FIELD]

The present invention relates to a technique for transmitting an examination order to a modality to be used in a medical examination.

### [BACKGROUND ART]

The order of an endoscopic examination (hereinafter, also referred to as an "examination order") is generated in a hospital information system such as an ordering system, and is issued to an endoscopy department system. The examination order includes order information on an endoscopic examination, such as scheduled time of examination start and examination end, patient identification information (patient ID), examination item, doctor in charge of examination, and examination room.

Endoscopic examinations have been conventionally performed, in which a doctor observes an endoscopic image by inserting the tip of an endoscope connected to an endoscopic observation device into a patient body. Additionally, endoscopic ultrasonography (EUS), in which ultrasonic examination is performed from the inside of a digestive tract, not from the body surface, has been put into practical use with an endoscope including an ultrasonic device. Further, in a radiology department, a radiological examination by a radiological examination device is performed as an in-vivo examination different from the endoscopic examination.

As described above, modalities for acquiring medical images have diversified, but in recent years, a complex examination, in which plural types of image observation are performed on a patient by utilizing differences in the characteristics of respective modalities, has been performed. An improvement in diagnostic accuracy can be achieved by taking advantage of the strength of each modality and complementing each other deficient characteristics in each modality.

In order to perform an examination, it is required as its premises that an examination order be generated in advance, and a modality executes processing based on the examination order by acquiring it. And, when a complex examination is performed, it is preferable that the examination images acquired in respective modalities are recorded in association with each other. For example, Patent Document 1 discloses a technique in which: first order information, to which first supplementary information specifying a second examination is added, is sent to a first examination device and second order information, to which second supplementary information specifying a first examination is added, is sent to a second examination device; and the image data and examination information acquired in the respective examinations are recorded in association with the first and second supplementary information, respectively.

### [RELATED ART DOCUMENT]

### [PATENT DOCUMENT]

[Patent Document 1] Japanese Patent Application Publication No. 2008-3783

### [DISCLOSURE OF THE INVENTION]

### [PROBLEM TO BE SOLVED BY THE INVENTION]

Conventionally, an examination order is assumed to be acquired and executed by one modality, and it is not assumed that one examination order is acquired and executed by a plurality of modalities. Therefore, in a conventional examination work support system, an examination order is generated for each examination in each modality, and also in Patent Document 1, a separate examination order is generated for each examination in each modality in a complex examination.

If one examination order is generated for a complex examination such that a plurality of modalities related to the complex examination can acquire the examination order, it is efficient because examination images and examination information acquired in each modality in the complex examination can be collected, but there is an increased risk that an unrelated modality may erroneously acquire the examination order. For example, an examination order, the examination item of which is a "routine examination", is intended to perform a single examination in which an examination is performed by one endoscopic observation device, but if this examination order is acquired and performed by two endoscopic observing devices, an examination is performed based on an erroneous examination order in at least one of the endoscopic observing devices.

Further, for example, an examination order, the examination item of which is "EUS", is intended to perform a complex examination in which examinations are performed by an endoscopic observation device and an ultrasonic observation device arranged in the same examination room, respectively; however, if the examination order is acquired and performed by two endoscopic observation devices, an examination is performed based on an erroneous examination order in at least one of the endoscopic observation devices, also in this case.

Therefore, when it is intended that one examination order can be acquired by a plurality of modalities, it is desirable to construct a system in which an examination order to be acquired and performed by specific related modalities is not acquired by an unrelated modality.

The present invention has been made in view of these situations, and a purpose of the invention is to provide a technique for efficiently transmitting an examination order to a modality to be used in an examination.

### [MEANS FOR SOLVING THE PROBLEM]

In order to solve the above problem, an examination work support system according to an embodiment of the present invention comprises: an examination item management unit that manages an examination item and the maximum number of modalities that can acquire an examination order including the examination item in association with each other; an order recording unit that records a plurality of examination orders each including an examination item; a receiving unit that receives a request for transmitting an examination order from a modality or an order request device that is a terminal device; an order retrieval unit that extracts an examination order to be transmitted from the plurality of examination orders recorded in the order recording unit; and an order transmission unit that transmits the extracted examination order to the order request device. When the number of modalities that acquired an examination order reaches the maximum number that can acquire the examination order, the order retrieval unit does not extract the examination order as an examination order to be transmitted to the order request device; on the other hand, when the number of modalities that acquired an examination order is less than the maximum number that can acquire the examination order, the order retrieval unit extracts the examination order as an examination order to be transmitted to the order request device.

Arbitrary combinations of the aforementioned constituting elements and implementations of the invention in the form of methods, devices, systems, recording media, computer programs and so forth may also be effective as additional modes of the present invention.

### [BRIEF DESCRIPTION OF THE DRAWINGS]

Fig. 1 is a view illustrating a configuration of an examination work support system according to an embodiment;
Fig. 2 is a view illustrating a flowchart in which a modality acquires an examination order;
Fig. 3 is a view illustrating a management table of examination items;
Fig. 4 is a view illustrating a management table of modalities;
Fig. 5 is a view illustrating the statuses of examination orders managed by an order status management unit;
Fig. 6 is a view illustrating a detailed flowchart of order retrieval processing;
Fig. 7 is a view illustrating a variation of the management table of modalities;
Fig. 8 is a view illustrating a configuration of an examination work support system according to a variation; and
Fig. 9 is a view illustrating another example of a flowchart in which a modality acquires an examination order.

### [MODE FOR CARRYING OUT THE INVENTION]

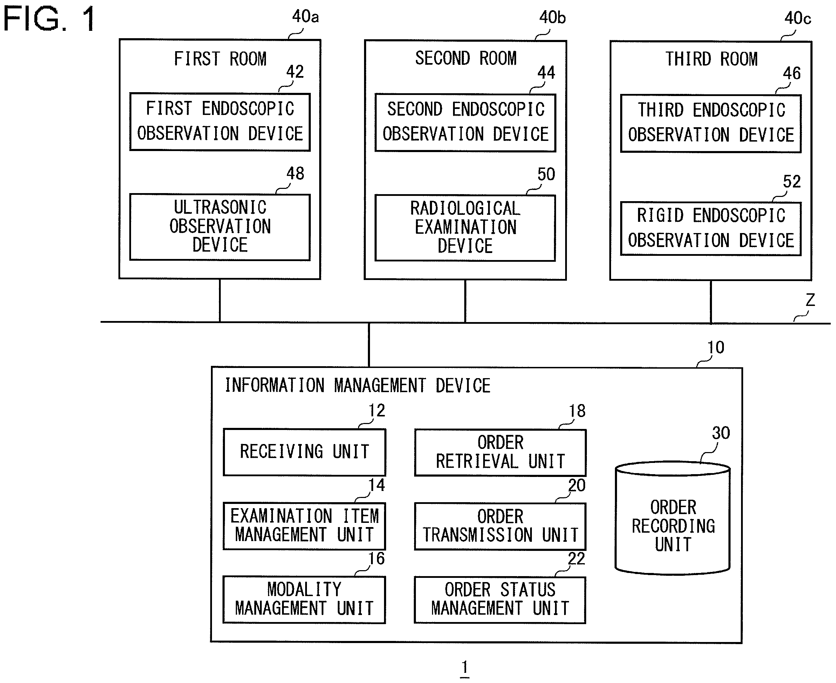

Fig. 1 is a view illustrating a configuration of an examination work support system 1 according to an embodiment of the present invention. The examination work support system 1 is a system for supporting examination work, in particular, endoscopic examination work, and includes a plurality of modalities and an information management device 10, in which each modality is connected to the information management device 10 by a network 2 such as LAN (local area network). The modality may be an examination device that acquires an examination image.

In the examination work support system 1, a first room 40a is an endoscopic examination room where a first endoscopic observation device 42 and an ultrasonic observation device 48 are arranged. A second room 40b is a radiological examination room where a second endoscopic observation device 44 and a radiological examination device 50 are arranged, and a third room 40c is an operating room where a third endoscopic observation device 46 and a rigid endoscopic observation device 52 are arranged. Each of the first endoscopic observation device 42, the second endoscopic observation device 44, the third endoscopic observation device 46, the ultrasonic observation device 48, the radiological examination device 50, and the rigid endoscopic observation device 52 is included in the examination work support system 1 as a modality for performing an examination.

Each of the first endoscopic observation device 42, the second endoscopic observation device 44, and the third endoscopic observation device 46 is an observation device for displaying an endoscopic image on a display, with a normal flexible endoscope being connected thereto. An endoscopic observation device is a basic observation device for performing an endoscopic examination, and a doctor can perform a normal examination by using this endoscopic observation device and can perform a complex examination by using other modalities as well.

The ultrasonic observation device 48 is an observation device for displaying an ultrasonic image on a display, with an ultrasonic endoscope being connected thereto. The inside of a tissue that cannot be seen with a normal endoscope can be observed with an ultrasonic endoscope, so that the depth of reach of a focus such as an ulcer, a submucosal tumor that cannot be seen on the surface, and the like can be examined.

The radiological examination device 50 is an examination device for imaging the structure of a bone, tissue, organ, or the like by using radiation. A doctor can examine the inside of the body of a patient in detail by performing a radiological examination in combination with a normal endoscopic examination.

The rigid endoscopic observation device 52 is an observation device for displaying a rigid endoscopic image on a display, with a rigid endoscope being connected thereto. A rigid endoscope is typically a laparoscope that is inserted into a body through a small hole opened in an abdomen such that diagnosis or surgery is performed, and may have a function of inserting a treatment tool or perfusing water.

As described above, the examination work support system 1 is configured to have a plurality of modalities. In order for a doctor to perform an examination using a modality, it is necessary that the modality should acquire an examination order by accessing the information management device 10 before the start of the examination.

The information management device 10 has a function of managing examination orders, and comprises a receiving unit 12, an examination item management unit 14, a modality management unit 16, an order retrieval unit 18, an order transmission unit 20, an order status management unit 22, and an order recording unit 30. In Fig. 1, each element described as a functional block performing various processing can be constituted by a circuit block, a memory, and other LSIs in terms of hardware and achieved by a program loaded in a memory, etc., in terms of software. Therefore, it is understood by those skilled in the art that these functional blocks can be achieved in various forms by hardware only, software only, or a combination thereof, and the blocks should not be limited to any one of them.

The order recording unit 30 records a plurality of examination orders each including an examination item. The examination order includes not only an examination item but also order information on an endoscopic examination, such as scheduled time of start and end of examination, patient identification information (patient ID), doctor in charge of examination, and room information. The order recording unit 30 may capture and record a plurality of examination orders in an endoscopy department system, which were generated in a hospital information system, but it may be a database which records a plurality of examination orders in the hospital information system.

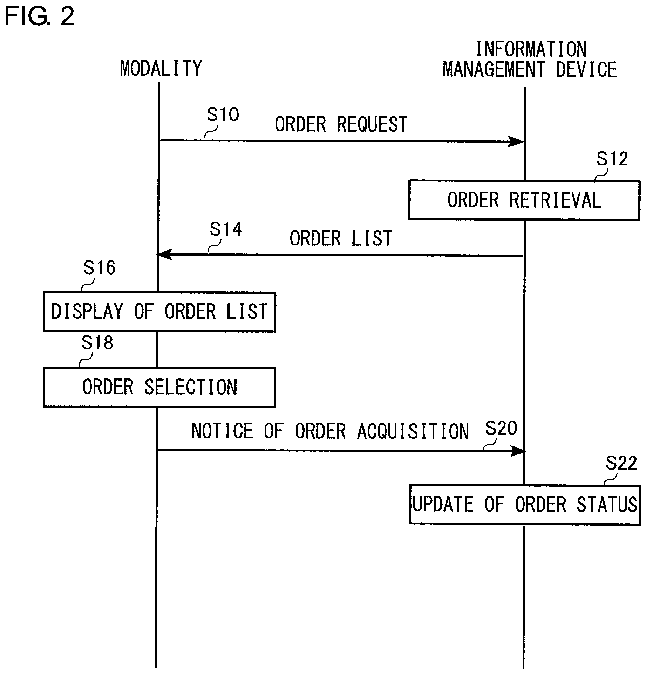

Fig. 2 illustrates a flowchart in which a modality acquires an examination order. A medical professional, such as a doctor, transmits an order request from a modality, an order request device, to the information management device 10 (S10). When the receiving unit 12 receives the request for transmitting an examination order from the modality in the information management device 10, the order retrieval unit 18 extracts examination orders to be transmitted by retrieving a plurality of examination orders recorded in the order recording unit 30 (S12). The order transmission unit 20 transmits a list of the examination orders extracted by the order retrieval unit 18 to the modality (S14).

When receiving the order list, the modality which is an order request device displays the order list on a display connected to the modality (S16). When a medical professional selects one examination order from the order list (S18), the modality transmits an acquisition notice of the selected examination order to the information management device 10 (S20). When the receiving unit 12 receives the acquisition notice in the information management device 10, the order status management unit 22 registers as a status of the examination order that the examination order was acquired by the modality (S22). Thereby, it is managed that the examination order was acquired by the modality. The order status management unit 22 may manage the status of the examination order in detail by setting it as "in preparation" before the examination starts and as "under examination" when the examination starts, but in the embodiment, the status may be managed such that it can be confirmed which modality acquired the examination order.

In the examination work support system 1 of the embodiment, one examination order is generated for a complex examination performed by using a plurality of modalities for one patient. The plurality of modalities related to the complex examination acquire the same examination order and record images and examination information captured in association with the examination order. With a plurality of examinations being associated with one examination order, as described above, it becomes possible to collect the examination images and examination information acquired in the respective examinations into one group, thereby allowing examination results to be managed efficiently.

On the other hand, if one examination order is allowed to be acquired by a plurality of modalities, there is an increased risk that an unrelated modality may erroneously acquire the examination order. As illustrated in Fig. 2, a modality selects and acquires the examination order to be performed from now on, from the order list that has been provided by the information management device 10. Therefore, if the order list does not include an examination order not to be performed in a modality, the information management device 10 can reduce the risk that a modality may erroneously select the examination order. As described above, the information management device 10 of the embodiment reduces the possibility that a modality may erroneously acquire an examination order by generating an order list according to a predetermined condition. The order list may be provided from the information management device 10 to a modality, as described later, but the list may be provided to another terminal device such that the order selected by the another terminal device is transmitted to a modality.

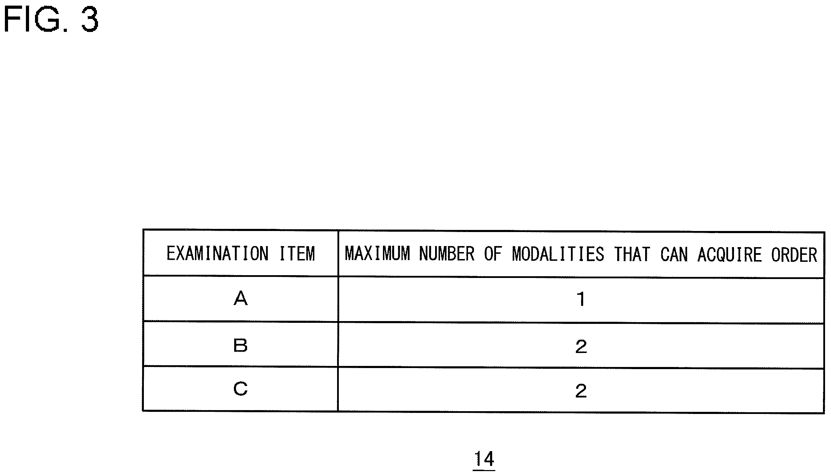

The details of the examination order management performed by the above information management device 10 will be described. Fig. 3 illustrates a management table of examination items. The examination item management unit 14 manages, in the management table, an examination item and the maximum number of modalities that can acquire an examination order including the examination item in association with each other. In this management table, it is registered that: an examination order including an examination item A can be acquired by up to one modality; an examination order including an examination item B can be acquired by up to two modalities; and an examination order including an examination item C can be acquired by up to two modalities.

For example, in the examination item A that is an upper normal examination (upper routine examination), an examination is to be performed by connecting a normal endoscope to an endoscopic observation device. In the examination item B that is an ultrasonic endoscopic examination, both an examination performed by connecting a normal endoscope to an endoscopic observation device and an examination performed by connecting an ultrasonic endoscope to an ultrasonic observation device are to be performed. In the examination item C that is ERCP (endoscopic retrograde cholangiopancreatography), both an examination performed by connecting a normal endoscope to an endoscopic observation device and a radiological examination in which a contrast medium is inserted from the catheter of an endoscope to capture X-ray photographs of a pancreatic duct and a bile duct are to be performed.

The "maximum number that can acquire an order" indicates the maximum number of modalities that can acquire one examination order, and the examination order is prohibited from being acquired by the number of modalities more than this maximum number. For example, it is determined that an examination order including the examination item B is acquired by up to two modalities, and hence when the examination order is already acquired by two modalities, it is managed that an order list to be transmitted in response to an order request from a modality should not include the examination order. On the other hand, when an examination order including the examination item B is acquired by one modality, the examination order may be transmitted by being included in an order list on the condition that other requirements are met.

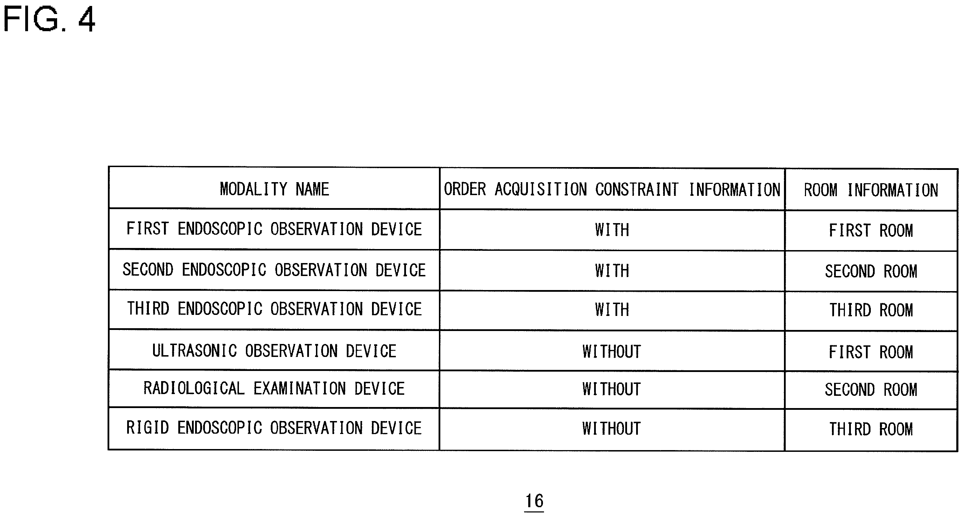

Fig. 4 illustrates a management table of modalities. In the management table, the modality management unit 16 manages a modality in association with order acquisition constraint information. The order acquisition constraint information is used to determine, when a modality acquires an examination order, whether there is any constraint in relation to other modalities. Herein, the modality management unit 16 further manages a modality also in association with information on a room where the modality is arranged.

When the order acquisition constraint information on a modality indicates "constrained", and in acquiring an examination order, when the examination order has already been acquired by a modality on which the order acquisition constraint information indicates "constrained", the examination order cannot be acquired. That is, an examination order is acquired only by one modality on which the order acquisition constraint information indicates "constrained", and should not be acquired by two or more modalities on each of which the order acquisition constraint information indicates "constrained."

When order acquisition constraint information on a modality indicates "not constrained", the modality can acquire an examination order irrespective of the type of a modality that has already acquired the examination order. When the number of modalities that acquired an examination order reaches the "maximum number" illustrated in Fig. 3, the examination order cannot be acquired, as described above.

In the management table of modalities illustrated in Fig. 4, the order acquisition constraint information on each of the first endoscopic observation device 42, the second endoscopic observation device 44, and the third endoscopic observation device 46 is set to be "constrained", and that on each of the modalities other than them, that is, that on each of the ultrasonic observation device 48, the radiological examination device 50, and the rigid endoscopic observation device 52 is set to be "not constrained."

There is virtually almost none of the case where multiple endoscopic observation devices are used in one endoscopic examination. Therefore, in the management table illustrated in Fig. 4, the order acquisition constraint information on the endoscopic observation devices are set to be "constrained", so that the multiple endoscopic observation devices do not acquire the same examination order. For example, when the first endoscopic observation device 42 has acquired a certain examination order, the second endoscopic observation device 44 or the third endoscopic observation device 46 never acquires the same examination order. That is, when a certain examination order has been acquired by the first endoscopic observation device 42, it is managed that an order list to be transmitted in response to an order request from the second endoscopic observation device 44 or the third endoscopic observation device 46 does not include the examination order, so that the second endoscopic observation device 44 and the third endoscopic observation device 46 cannot acquire the examination order.

On the other hand, even when a certain examination order has been acquired by the first endoscopic observation device 42, the examination order may be included in an order list to be transmitted to the ultrasonic observation device 48, the radiological examination device 50, and the rigid endoscopic observation device 52, the order acquisition constraint information on each of which is set to be "not constrained."

The order status management unit 22 manages the status of an examination order. Herein, the order status management unit 22 manages, as a status, at least the information indicating whether an examination order has been acquired by a modality, and when acquired, also manages the information indicating which modality has acquired.

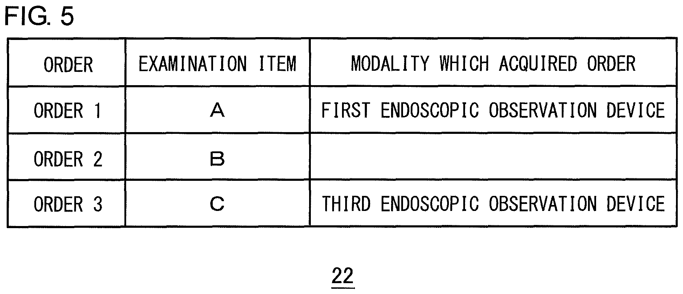

Fig. 5 illustrates an example of the statuses of the examination orders managed by the order status management unit 22. An order 1 includes the examination item A as order information, and has been acquired by the first endoscopic observation device 42. An order 2 includes the examination item B as order information, and has not been acquired by a modality yet. An order 3 includes the examination item C as order information, and has been acquired by the third endoscopic observation device 46.

Hereinafter, a method will be described, in which the order retrieval unit 18 generates an order list to be transmitted to a modality. Retrieval processing by the order retrieval unit 18 is performed in the step of S12 in Fig. 2. With reference to Fig. 2, when the receiving unit 12 receives a request for transmitting an examination order from a modality (S10), the order retrieval unit 18 performs retrieval processing for extracting an examination order to be transmitted from a plurality of examination orders recorded in the order recording unit 30 (S12).

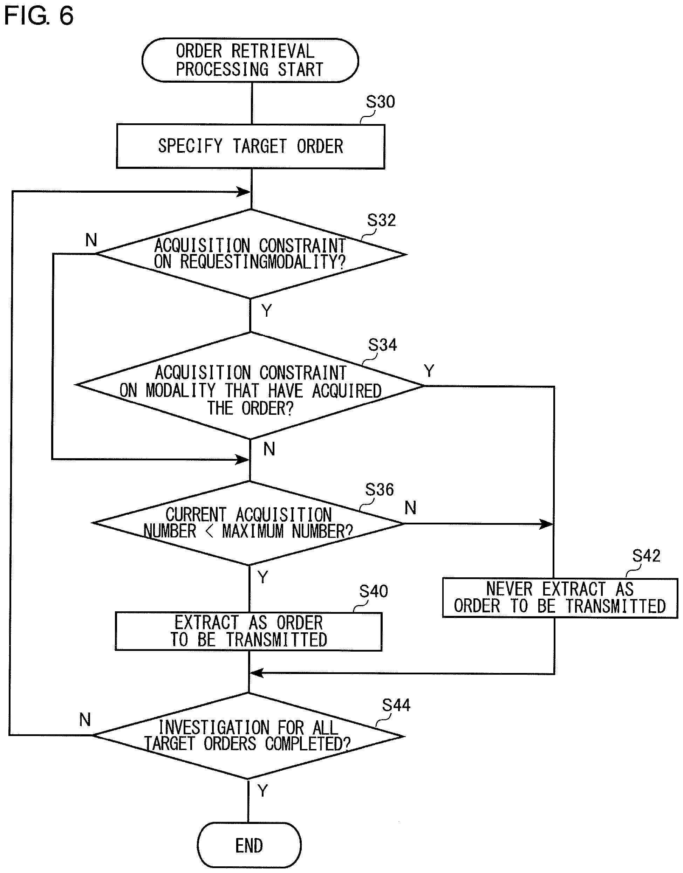

Fig. 6 illustrates a detailed flowchart of the order retrieval processing. The order retrieval unit 18 specifies an examination order to be transmitted from the order recording unit 30 based on the retrieval condition included in the request for transmitting an examination order (S30). For example, when the transmission request includes a condition for specifying examination orders not yet performed for today, the order retrieval unit 18 specifies, as candidates to be transmitted, the examination orders not yet performed for today from the order recording unit 30. Hereinafter, it is investigated whether each of the examination orders that are candidates to be transmitted may be transmitted to a modality.

First, the order retrieval unit 18 determines whether the modality that has transmitted the request for transmitting an examination order is a modality managed, by the modality management unit 16, in association with the order acquisition constraint information indicating that there is a constraint (S32). Specifically, with reference to the management table of Fig. 4, it is determined whether the modality that has transmitted the request is an endoscopic observation device.

When the modality that has transmitted the request is a modality having an order acquisition constraint (S32/Y), the order retrieval unit 18 determines whether a modality having an order acquisition constraint is included in the modalities that have already acquired an examination order to be investigated (S34). That is, when the modality that has transmitted the request is an endoscopic observation device, it is determined whether the examination order under investigation has already been acquired by another endoscopic observation device. If a modality indicating that there is a constraint on the order acquisition constraint information is included in the modalities that have acquired the examination order (S34/Y), the order retrieval unit 18 does not extract the examination order as an examination order to be transmitted to the modality (S42). This is done to avoid the risk that the examination order may be erroneously acquired in the modality that has transmitted the request.

On the other hand, if a modality indicating that there is a constraint on the order acquisition constraint information is not included in the modalities that have acquired the examination order (S34/N), the order retrieval unit 18 extracts the examination order as an examination order to be transmitted to the modality on condition that the number limitation is met in S36 (S40). The step of S36 will be described.

With reference to the management table illustrated in Fig. 3, the order retrieval unit 18 acquires the maximum number that can acquire the order from the examination item included in the examination order, and also with reference to the order statuses (see Fig. 5) managed by the order status management unit 22, acquires the number of the modalities that have acquired the examination order. The order retrieval unit 18 compares the maximum number with the current acquisition number (S36), and if the number of the modalities that have acquired the examination order reaches the maximum number that can acquire the examination order (S36/N), the order retrieval unit 18 does not extract the examination order as an examination order to be transmitted to the modality (S42); on the other hand, if the number of the modalities that have acquired the examination order is less than the maximum number that can acquire the examination order (S36/Y), the order retrieval unit 18 extracts the examination order as an examination order to be transmitted to the modality (S40).

In the step of S36, it is managed that an examination order is acquired by modalities the number of which is less than or equal to the maximum number set in an examination item, as described above. If an examination order is acquired by modalities the number of which is more than the maximum number, there is a situation where a modality erroneously acquires the examination order. Therefore, in order to avoid such a situation, the order retrieval unit 18 does not include an examination order, which has been acquired by modalities the number of which reaches the maximum number, in an order list such that the examination order is not acquired by a modality.

In the step of S32, when the modality that has transmitted a request is not a modality having an order acquisition constraint (S32/N), the order retrieval unit 18 skips the step of S34 to perform the step of S36. The order retrieval processing is performed until the investigation of all of the examination orders specified in the step of S30 is completed (S44/N), and the processing is ended when tthe investigation is completed (S44/Y). All of the examination orders extracted as an examination order to be transmitted are collected, and the order transmission unit 20 transmits the extracted examination orders to the modality that has transmitted the request for transmitting an examination order (S14 in Fig. 2).

Hereinafter, description will be made by using specific examples. The statuses of the orders 1 to 3 are made the same as those in Fig. 5.

### <Specific Example 1: Case where Second Endoscopic Observation Device 44 Transmits Order Request>

Because the second endoscopic observation device 44 is a modality managed in association with order acquisition constraint information indicating that there is a constraint (S32/Y), and because the orders 1 and 3 are acquired by the first endoscopic observation device 42 and the third endoscopic observation device 46 that are managed in association with the order acquisition constraint information indicating that there is a similar constraint, respectively (S34/Y), the orders 1 and 3 are not extracted as an examination order to be transmitted (S42). Meanwhile, because the order 2 has been acquired by no modality (S34/N), and because the number of the modalities that have acquired the order 2 does not reach the maximum number (2) that can acquire the order 2 (S36/Y), the order 2 is extracted as an examination order to be transmitted (S40). Therefore, the order transmission unit 20 transmits the order 2 to the second endoscopic observation device 44.

### <Specific Example 2: Case where Ultrasonic Observation Device 48 Transmits Order Request>

Because the ultrasonic observation device 48 is a modality managed in association with order acquisition constraint information indicating that there is no constraint (S32/N), an order, in which a number limitation is met, is extracted as an examination order to be transmitted (S36/Y). Herein, the maximum number of the order 1 including the examination item A is one and it has already been acquired by the first endoscopic observation device 42 (S36/N), and hence the order 1 is not extracted as an examination order to be transmitted (S42). On the other hand, the number of the modalities that have acquired either the order 2 or the order 3 does not reach the maximum number (S36/Y), and hence the orders 2 and 3 are extracted as an examination order to be transmitted (S 40). Therefore, the order transmission unit 20 transmits them to the ultrasonic observation device 48. Similarly, when the radiological examination device 50 or the rigid endoscopic observation device 52 transmits an order request, the order transmission unit 20 transmits the orders 2 and 3.

In the management table illustrated in Fig. 4, the modality management unit 16 manages a modality in association with information on a room where the modality is arranged. The order transmission unit 20 transmits the examination order extracted by the order retrieval unit 18 to the modality that has transmitted a request for transmitting an examination order, and may transmit at least part of the examination order extracted by the order retrieval unit 18 to another modality arranged in the same room as that of the modality.

In the specific example 1 described above, it has been described that when the second endoscopic observation device 44 transmits an order request, the order transmission unit 20 transmits the order 2 to the second endoscopic observation device 44 by the retrieval processing illustrated in Fig. 6. At this time, the order transmission unit 20 retrieves a modality arranged in the same room as that of the second endoscopic observation device 44 by referring to the room information of the management table illustrated in Fig. 4, so that all or part of the examination order extracted by the order retrieval unit 18 is transmitted also to the modality in the same room. This is because multiple modalities in the same room are not simultaneously used in different complex examinations and are highly likely to be used in the same complex examination. Because the modality arranged in the same room as that of the second endoscopic observation device 44 is the radiological examination device 50, the order transmission unit 20 may transmit the order 2 to the radiological examination device 50. Thereby, multiple modalities arranged in the same room are provided with an option for selecting the same examination order, and hence it becomes possible to reduce the risk that each modality may erroneously acquire an examination order.

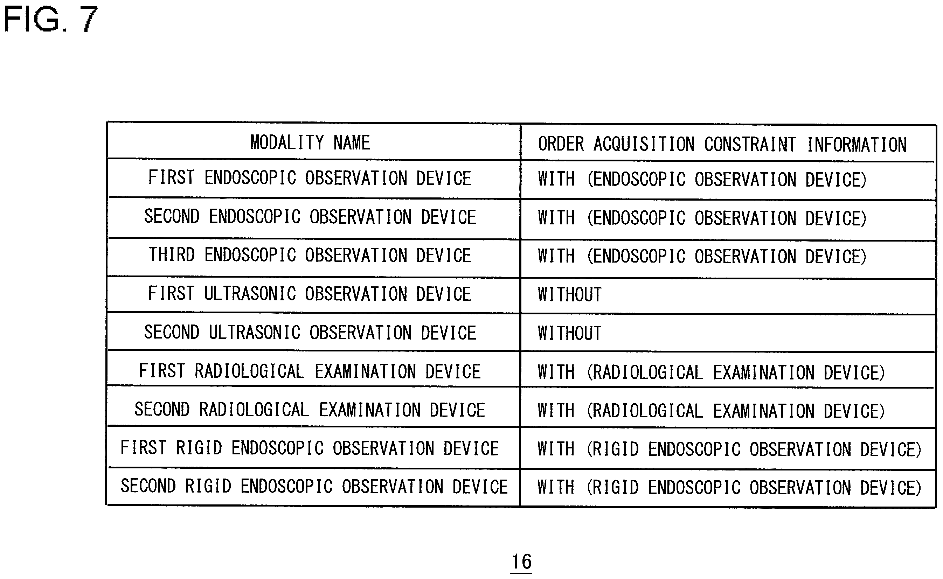

Fig. 7 illustrates a variation of the management table of modalities. In the management table illustrated in Fig. 7, the order acquisition constraint information is set as information for determining whether, when a modality acquires an examination order, there is a constraint in relation to a modality of the same type. In a complex examination, a plurality of examinations may be simultaneously performed by using modalities of different types, but it is extremely rare for modalities of the same type to be used. Therefore, by setting the order acquisition constraint information as information for determining whether there is a constraint in relation to a modality of the same type, it becomes possible to avoid the risk that one examination order may be acquired by a plurality of modalities of the same type.

In the management table illustrated in Fig. 7, not only acquisition of one examination order by a plurality of endoscopic observation devices is prohibited, but also acquisition by a plurality of radiological examination devices is prohibited, and acquisition by a plurality of rigid endoscopic observation devices is also prohibited.

The present invention has been described above based on an embodiment. The embodiment is described for exemplary purposes only, and it can be readily understood by those skilled in the art that various modifications may be made by making various combinations of the aforementioned components or processes, which are also encompassed by the scope of the present invention.

For example, the modality management unit 16 may manage a modality in association with an examination item by which an examination order can be acquired. For example, the radiological examination device 50 is used in ERCP, but never used in an ultrasonic endoscopic examination. Therefore, when an order request is transmitted from the radiological examination device 50, and when the order retrieval unit 18 includes an order for an ultrasonic endoscopic examination in an order list, a risk consequently occurs, in which the order for an ultrasonic endoscopic examination may be erroneously acquired. Therefore, it is preferable that the order retrieval unit 18 reduces the possibility that a modality may erroneously acquire an order, by extracting an examination order including an examination item associated with the modality.

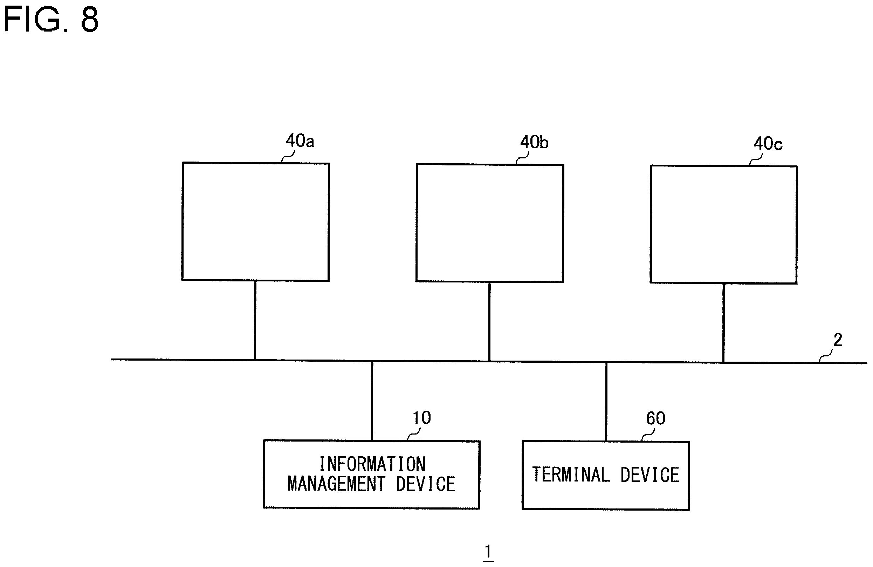

Fig. 8 is a view illustrating a configuration of an examination work support system 1 according to a variation. The examination work support system 1 according to the variation comprises: a plurality of modalities; an information management device 10; and a terminal device 60, and each modality is connected to the information management device 10 and the terminal device 60 by a network 2, such as LAN (Local Area Network). Each modality may be an examination device that acquires an examination image. As described in the embodiment, the first endoscopic observation device 42 and the ultrasonic observation device 48 are arranged in the first room 40a, and the second endoscopic observation device 44 and the radiological examination device 50 are arranged in the second room 40b, and the third endoscopic observation device 46 and the rigid endoscopic observation device 52 are arranged in the third room 40c.

For example, the terminal device 60 is arranged in an endoscopy department, etc., and is operated by a medical professional, such as a doctor. The terminal device 60 may be arranged in each examination room and operated by a medical professional. In the embodiment, a modality transmits a request for transmitting an examination order to the information management device 10, but in the variation, the terminal device 60 operates as an order request device that transmits a request for transmitting an examination order to the information management device 10 and receives an order list. Also in the variation, the information management device 10 has the configuration described in the embodiment.

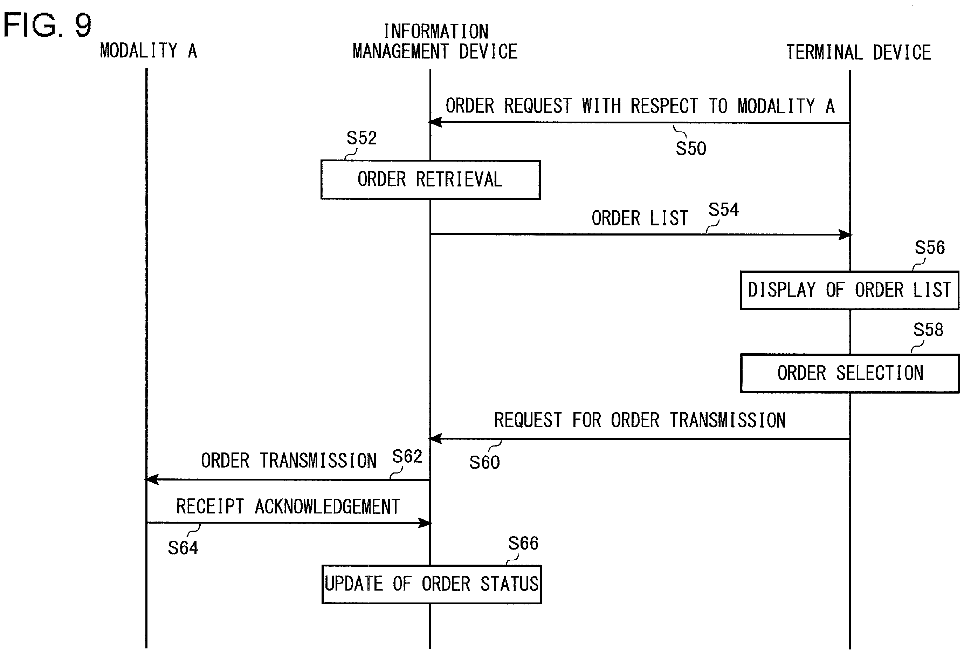

Fig. 9 illustrates another example of the flowchart in which a modality acquires an examination order. In this variation, the terminal device 60 receives an order list and selects an examination order, and the selected examination order is transmitted from the information management device 10 to a modality such that it is acquired by the modality.

A medical professional, such as a doctor, transmits an order request from the terminal device 60 to the information management device 10 (S50). Herein, the order request requests transmission of an examination order with respect to a specific modality, and in this variation, an order request with respect to a modality A is transmitted from the terminal device 60 to the information management device 10.

In the information management device 10, when the receiving unit 12 receives the request for transmitting an examination order with respect to the modality A from the terminal device 60, the order retrieval unit 18 extracts examination orders to be transmitted by retrieving the plurality of examination orders recorded in the order recording unit 30 (S52). The order transmission unit 20 transmits a list of the examination orders extracted by the order retrieval unit 18 to the terminal device 60 (S54).

When receiving the order list with respect to the modality A, the terminal device 60 displays the order list on a display connected to the terminal device 60 (S56). When a medical professional selects one examination order from the order list (S58), the terminal device 60 transmits, to the information management device 10, a request for transmitting an order that is for providing the selected examination order to the modality A (S60). In the information management device 10, when the receiving unit 12 receives the transmission request, the order transmission unit 20 transmits the examination order selected in the terminal device 60 to the modality A (S62). When receiving the examination order, the modality A transmits a receipt acknowledgement to the information management device 10 (S64), and in response to this, the order status management unit 22 registers, as the status of the examination order, the fact that the examination order has been acquired by the modality A (S66). Thereby, it is managed that the examination order has been acquired by the modality. The order status management unit 22 may manage the status of an examination order in detail by indicating as "In preparation" before the examination starts and "Under examination" when the examination starts.

Thus, the examination work support system 1 of the variation is different from that of the embodiment in that the processing for selecting an order is performed by the terminal device 60 side, not by a modality side; but other points are the same. That is, in the variation, an order selection operation is performed in the terminal device 60, and the selected order is transmitted to the corresponding modality. For example, when the terminal device 60 is provided for each examination room, the examination order selected by the terminal device 60 may be transmitted to the corresponding modality by matching the terminal device 60 and the modality.

### [DESCRIPTION OF REFERENCE NUMERALS]

- 1: EXAMINATION WORK SUPPORT SYSTEM
- 2: NETWORK
- 10: INFORMATION MANAGEMENT DEVICE
- 12: RECEIVING UNIT
- 14: EXAMINATION ITEM MANAGEMENT UNIT
- 16: MODALITY MANAGEMENT UNIT
- 18: ORDER RETRIEVAL UNIT
- 20: ORDER TRANSMISSION UNIT
- 22: ORDER STATUS MANAGEMENT UNIT
- 30: ORDER RECORDING UNIT
- 40a: FIRST ROOM
- 40b: SECOND ROOM
- 40c: THIRD ROOM
- 42: FIRST ENDOSCOPIC OBSERVATION DEVICE
- 44: SECOND ENDOSCOPIC OBSERVATION DEVICE
- 46: THIRD ENDOSCOPIC OBSERVATION DEVICE
- 48: ULTRASONIC OBSERVATION DEVICE
- 50: RADIOLOGICAL EXAMINATION DEVICE
- 52: RIGID ENDOSCOPIC OBSERVATION DEVICE
- 60: TERMINAL DEVICE

### [INDUSTRIAL APPLICABILITY]

The present invention can be used in a technical field in which an examination order is dealt with.

## Claims

1. An examination work support system comprising:
an examination item management unit that manages an examination item and the maximum number of modalities that can acquire an examination order including the examination item in association with each other;
an order recording unit that records a plurality of examination orders each including an examination item;
a receiving unit that receives a request for transmitting an examination order from an order request device that is a modality or a terminal device;
an order retrieval unit that extracts an examination order to be transmitted from the plurality of examination orders recorded in the order recording unit; and
an order transmission unit that transmits the extracted examination order to the order request device, wherein
when the number of modalities that have acquired an examination order reaches the maximum number of modalities that can acquire the examination order, the order retrieval unit does not extract the examination order as an examination order to be transmitted to the order request device;
on the other hand, when the number of modalities that have acquired an examination order is less than the maximum number of modalities that can acquire the examination order, the order retrieval unit extracts the examination order as an examination order to be transmitted to the order request device.

2. The examination work support system according to claim 1 further comprising:
a modality management unit that manages a modality in association with order acquisition constraint information, wherein
the order acquisition constraint information is used to determine, when a modality acquires an examination order, whether there is any constraint in relation to other modalities, and wherein
when the receiving unit receives, from the order request device, a request for transmitting an examination order with respect to a modality managed in association with order acquisition constraint information indicating that there is a constraint, the order retrieval unit does not extract the examination order as an examination order to be transmitted to the order request device if a modality indicating that there is a constraint on order acquisition restriction information is included in the modalities that have acquired the examination order;
on the other hand, the order retrieval unit extracts the examination order as an examination order to be transmitted to the order request device if a modality indicating that there is a constraint on order acquisition restriction information is not included in the modalities that have acquired the examination order.

3. The examination work support system according to claim 2, wherein
the modality management unit manages a modality in association with a room where the modality is arranged, and wherein
the order transmission unit transmits an examination order with respect to the modality extracted by the order retrieval unit to the order request device that has transmitted a request for transmitting the examination order, and transmits at least part of the examination order extracted by the order retrieval unit to another modality or terminal device arranged in the same room as that of the modality.

4. The examination work support system according to claim 2, wherein
the order acquisition constraint information determines whether, when a modality acquires an examination order, there is a constraint in relation to a modality of the same type.

5. The examination work support system according to claim 2, wherein
the modality management unit manages a modality in association with an examination item by which an examination order can be acquired.
